# EUROPEAN PATENT SPECIFICATION

(11) **EP 3 995 093 B1**
(45) Date of publication and mention of the grant of the patent: **06.08.2025**
(21) Application number: 20834386.3
(22) Date of filing: 29.06.2020
(51) Int. Cl.: A61B 17/70

(54) **CORRECTION DEVICE**
KORREKTURVORRICHTUNG
DISPOSITIF DE CORRECTION

(30) Priority: 01.07.2019 JP 2019122920
(43) Date of publication of application: 11.05.2022
(73) Proprietor: Mizuho Corporation, Bunkyo-ku Tokyo 113-0033 (JP)
(72) Inventor: EBARA Sohei, Fujisawa-shi, Kanagawa 251-0041 (JP)
(74) Representative: SSM Sandmair
(86) International application number: PCT/JP2020/025521
(87) International publication number: WO 2021/002323

(56) References cited:
- EP-A1- 3 254 634
- US-A1- 2012 203 279
- US-A1- 2014 316 475
- US-A1- 2014 350 612
- US-A1- 2014 364 912
- US-A1- 2018 228 518

## Description

### Technical Field

The present invention relates to a corrective appliance for assisting in correction and fusion during implementation of spinal deformity correction and fusion surgery in which spinal deformity is subjected to the correction and fusion by a spinal deformity correction and fusion system including a vertebra fixing tool to be fixed to each of a plurality of vertebrae and a rod member to be coupled to the vertebra fixing tool.

### Background Art

In a normal condition of a spine, the spine is generally straight when viewed from the back, cervical vertebrae and lumbar vertebrae curve forward and thoracic vertebrae and sacral vertebrae curve backward when viewed from the side. In this way, the spine shows an approximately S-shaped appearance. Spinal deformity causing abnormality at the spine is a disease with the deformed spine, and includes scoliosis, kyphosis, and kyphoscoliosis, for example. Scoliosis is a disease in which the spine is twisted while being curved laterally. Kyphosis is a disease in which the angle of thoracic kyphosis becomes extremely large, or lumbar lordosis is lost to be deformed toward kyphosis. Kyphoscoliosis is a disease caused by a combination of scoliosis and kyphosis.

For treatment of such types of spinal deformity, spinal deformity correction and fusion surgery is widely conducted. The spinal deformity correction and fusion surgery is an operation for correcting a deformed spine to a normal state or a state close to the normal state and then fixing the corrected spine using a spinal deformity correction and fusion system (self-contained member, which is what is called an implant) described later. Posterior correction and fusion surgery or anterior correction and fusion surgery is employed for such operation. In particular, the posterior correction and fusion surgery is conducted as follows. For implementation of the posterior correction and fusion surgery, a patient is positioned on an operating table in a prone position. Then, an operative wound or a percutaneous surgical wound using minimally invasive technique is placed along the median line of the patient's back, and posterior elements of the spine are unfolded. Subsequently, a spinal deformity correction and fusion system described later (see patent literature 1, for example) is mounted on the spine to three-dimensionally correct the spinal deformity. The spine is fixed in this state.

Generally, a system to be employed as the spinal deformity correction and fusion system includes: a plurality of screw members each to be screwed into a vertebral body through a pedicle of each vertebra of a spine; a hook member to be hooked on a pedicle, a vertebral arch, or a transverse process, for example, of each vertebra; rod members in a pair to be coupled to a top-opened groove of each screw and that of each hook member, extending along an axis direction of the spine, and arranged at an interval in a crosswise direction of a patient, etc.

For example, when a patient with scoliosis is subjected to posterior correction and fusion surgery conducted to correction and fusion of spinal deformity by mounting the above-described spinal deformity correction and fusion system on a spine, the screw member and the hook member are first fixed to each of a plurality of vertebrae to be corrected. Next, the rod member is engaged with the top-opened groove of the screw member and that of the hook member. At this time, the rod member extends linearly while the spine is deformed to make it quite difficult to engage the rod member with the top-opened groove of the screw member and that of hook member. In response to this, an operator uses a dedicated surgical instrument to give a curve to the rod member along the scoliosis deformity of the spine. Then, the curved rod is engaged with the top-opened groove of the screw member and that of the hook member fixed to each vertebra. Then, in order to prevent the rod member from coming off the top-opened groove of each screw member and that of each hook member, set screws are temporarily tightened in these top-opened grooves.

Next, the outer peripheral surface of the rod member is sandwiched with a dedicated surgical instrument corresponding to a pair of pliers and this surgical instrument is rotated about 90° to rotate the rod member about 90° about its axis. In this way, operation of correcting the scoliosis deformity including twisting of the spine is performed. Operation of correcting the scoliosis deformity of the spine is also performed by applying compressive load or tensile load between members such as a plurality of screw members arranged in the axis direction (cranio-caudal direction) of the spine and screw members arranged adjacent to each other in the axis direction of the spine, for example, using a dedicated surgical instrument. After implementation of such operation for the correction, the set screws are fully tightened to form firm coupling of the rod member with each screw member and each hook member, thereby correcting and fixing the spine.

### Citation List

### Patent Literatures

Patent Literature 1: Japanese Patent Application Publication No. 2012-213625 US 2018/228518 A1 discloses tandem rod connectors and related methods.
EP 3 254 634 A1 discloses an orthopedic rod-to-rod connector and rod-to-rod connector assemblies for spinal fusion surgery.

### Summary of Invention

### Problem to be Solved by Invention

According to the conventional correction and fusion method using the spinal deformity correction and fusion system, the rod member is engaged with the top-opened groove of each screw member and that of each hook member while being curved along the scoliosis deformity of the patient. As the spine is under the scoliosis deformity including twisting, however, it becomes quite difficult to engage the rod member, even in the curved state, with the top-opened groove of each screw member and that of the hook member. Additionally, as a range of the correction and fusion using the spinal deformity correction and fusion system becomes longer, it becomes more difficult to engage the rod member with the top-opened groove of each screw member and that of the hook member.

According to the conventional correction and fusion method using the spinal deformity correction and fusion system, operation of correcting the scoliosis deformity including twisting of the spine is performed by sandwiching the outer peripheral surface of the rod member firmly with a dedicated surgical instrument corresponding to a pair of pliers (such as a rod gripper) and by rotating the rod member about 90°. If the rod member curved along the scoliosis deformity of the patient is rotated about 90° about its axis, however, this curvature of the rod member is replaced with backward curvature and forward curvature of the patient. However, as this curvature of the rod member does not conform to the physiological backward curvature and forward curvature of the patient, another trouble might be caused.

Furthermore, according to the conventional correction and fusion method using the spinal deformity correction and fusion system, the outer peripheral surface of the rod member is sandwiched firmly with a dedicated surgical instrument corresponding to a pair of pliers and is rotated 90°. This causes a risk of damage on the outer peripheral surface of the rod member at positions sandwiched with the surgical instrument corresponding to a pair of pliers. Additionally, as the set screws are temporarily tightened in the top-opened groove of each screw member and that of each hook member, rotating the rod member also causes a risk of damage on the outer peripheral surface of the rod member at positions of contact with the set screw. As a result, many of such damaged positions become causes for breakage of the rod member after indwelling inside a body.

The present invention has been made in view of the foregoing issues, and is intended to provide a corrective appliance capable of properly assisting in correction of spinal deformity by a spinal deformity correction and fusion system while limiting damage on a rod member when the spinal deformity is subjected to the correction and fusion by the spinal deformity correction and fusion system.

### Means of Solving Problem

The present invention is defined in claim 1 while preferred embodiments are set forth in the dependent claims.

Associated methods are also described herein to aid understanding the invention. These methods do not form part of the claimed invention The embodiments and/or examples of the following description which are not covered by the claims, are provided for illustrative purpose only and are only intended to assist the reader in understanding the present invention. However, such embodiments and/or examples which are not covered by the claims do not form part of the present invention that is solely defined by the claims. The aspects of the invention described below exemplify the configuration of the present invention, and are described separately by items in order to facilitate the understanding of various configurations of the present invention.
(1) A corrective appliance is used for assisting in correction and fusion when spinal deformity is subjected to the correction and fusion by a spinal deformity correction and fusion system including a vertebra fixing tool to be fixed to each vertebrae of a spine and a rod member to be coupled to the vertebra fixing tool. The corrective appliance includes: a clamp member to be attached detachably to the vertebra fixing tool and to be arranged close to the vertebra fixing tool; and a corrective member to be attached detachably to the clamp member and to be used for correcting the spinal deformity.

When the spinal deformity is subjected to the correction and fusion by the spinal deformity correction and fusion system, the corrective appliance described in the item (1) is capable of assisting in the correction using the clamp member and the corrective member. Specifically, after the vertebra fixing tool is fixed to each vertebra, the clamp member is attached to each vertebra fixing tool and the corrective member is attached further to each clamp member. Next, the corrective member is operated to correct the spinal deformity properly though each clamp member and each vertebra fixing tool and the corrected state is retained. Next, the rod member is coupled to each vertebra fixing tool to fix a spine in the corrected state. Then, the corrective member and each clamp member are detached from each vertebra fixing tool and taken out of a body. During the correction and fusion of the spinal deformity in this way by the spinal deformity correction and fusion system, unlike in the conventional case, it is not necessary to engage the rod member of the spinal deformity correction and fusion system with each vertebra fixing tool fixed to each vertebra under scoliosis deformity including twisting, or to perform operation of giving a curve to the rod member of the spinal deformity correction and fusion system using a dedicated surgical instrument or operation of firmly sandwiching the rod member and rotating (turning) the rod member using a dedicated surgical instrument. As a result, it becomes possible to limit damage on the rod member as a self-contained member. Furthermore, using the corrective appliance allows the rod member of the spinal deformity correction and fusion system to be easily engaged with (coupled to) each vertebra fixing tool. As a result, correction of the spinal deformity becomes easier to facilitate implementation of a surgery itself, thereby contributing to reduction in a duration of the surgery.

The corrective appliance described in the item (1) is used effectively, particularly if the correction and fusion by the spinal deformity correction and fusion system covers a long range. Specifically, if the range of the correction and fusion by the spinal deformity correction and fusion system is long, the clamp member is attached selectively to the vertebra fixing tool fixed to each vertebra, the corrective member is attached to each clamp member, a deformed spine is corrected partially, and the corrected state is retained using these clamp member and corrective member. Then, the rod member is coupled to each vertebra fixing tool. By doing so, the spine can be fixed in the corrected state.

(2) The corrective appliance described in the item (1) is characterized in that the clamp member includes a groove, and the corrective member is an auxiliary rod member configured to be engaged with the groove of the clamp member.

In the corrective appliance described in the item (2), the auxiliary rod member can be given a curve along scoliosis deformity and can be engaged with the groove of each clamp member fixed to each vertebra, instead of the rod member of the spinal deformity correction and fusion system (self-contained member). The auxiliary rod member can be used for correcting spinal deformity through the clamp member and the vertebra fixing tool. Then, the correction using the corrective appliance is completed and the correction is retained. In this state, the rod member as the self-contained member is coupled to each vertebra fixing tool, thereby allowing retention of the corrected state of the spinal deformity by the spinal deformity correction and fusion system. As a result, it becomes possible to limit damage on the rod member as the self-contained member, thereby reducing the occurrence of breakage of the rod member easily after the rod member indwells in a body.

(3) The corrective appliance described in the item (1) or (2) is characterized in that the clamp member includes supporting pieces in a pair movable closer to and farther from each other to support a head of the vertebra fixing tool in such a manner as to sandwich the head.

In the corrective appliance described in the item (3), the clamp member can be attached to support the head of the vertebra fixing tool in such a manner as to sandwich the head using the supporting pieces in a pair of the clamp member. Furthermore, a top-opened groove provided at the top face of the head of the vertebra fixing tool and used for engagement with the rod member can be exposed to the outside. As a result, after correction of spinal deformity and retention of the corrected state using the clamp member and the corrective member, the rod member can be engaged easily with the top-opened groove of each vertebra fixing tool. Furthermore, the vertebra fixing tool can be supported firmly from opposite lateral sides of the head using the supporting pieces in a pair of the clamp member. Thus, force of the correction applied from the clamp member can be transmitted properly to a vertebra through the vertebra fixing tool, making it possible to correct the spinal deformity properly.

(4) The corrective appliance described in the item (1) or (2) is characterized in that the clamp member includes a ring-like member to support a head of the vertebra fixing tool in such a manner as to surround an outer periphery of the head.

In the corrective appliance described in the item (4), the clamp member can be attached to support the head of the vertebra fixing tool in such a manner as to surround the outer periphery of the head using the ring-like member of the clamp member. This allows the head entirely of the vertebra fixing tool to be supported firmly using the ring-like member of the clamp member. Thus, force of the correction applied from the clamp member can be transmitted properly to a vertebra through the vertebra fixing tool, making it possible to correct spinal deformity properly.

(5) The corrective appliance described in any one of the items (2) to (4) is characterized in that the clamp member includes a fixing tool for fixing the auxiliary rod member to the groove of the clamp member.

In the corrective appliance described in the item (5), by operating the auxiliary rod member while the auxiliary rod member is temporarily tightened to the groove of each clamp member using the fixing tool, it becomes possible to correct spinal deformity easily through each clamp member. The auxiliary rod member is fixed firmly to the groove of each clamp member using the fixing tool. By doing so, the spinal deformity can be retained in the corrected state using the clamp member and the auxiliary rod member.

(6) The corrective appliance described in the item (4) or (5) is characterized in that the clamp member includes stopper pawl sections in a pair for sandwiching the head of the vertebra fixing tool.

In the corrective appliance described in the item (6), the stopper pawl sections in a pair can be used for fixing the clamp member to the vertebra fixing tool firmly. As a result, force of the correction applied from the clamp member can be transmitted properly to a vertebra through the vertebra fixing tool, making it possible to correct spinal deformity properly.

(7) The corrective appliance described in any one of the items (1) to (6) is characterized in that the corrective appliance is configured as a surgical instrument detachable from the vertebra fixing tool to be taken out of a body while the spinal deformity is corrected and fixed by the spinal deformity correction and fusion system.

As the corrective appliance described in the item (7) functions as a surgical instrument, it is not required to be made of a material having excellent biocompatibility such as titanium alloy or cobalt-chromium alloy. This imposes no limitation on a material to be used, leading to cost reduction.

(8) The corrective appliance described in any one of the items (2) to (7) is characterized in that the auxiliary rod member has an outer diameter same as that of the rod member.

The corrective appliance described in the item (8) can entirely be given a compact configuration.

(9) The corrective appliance described in any one of the items (6) to (8) is characterized in that the head of the vertebra fixing tool is provided with concaved sections for supporting tips of the stopper pawl sections.

In the corrective appliance described in the item (9), the stopper pawl sections of the clamp member are engaged with the concaved sections at the vertebra fixing tool. This makes it possible to minimize backlash of the clamp member relative to the vertebra fixing tool occurring during attachment of the clamp member to the vertebra fixing tool.

### Advantageous Effects of Invention

The corrective appliance according to the present invention is capable of properly assisting in correction of spinal deformity by a spinal deformity correction and fusion system while limiting damage on a rod member when the spinal deformity is subjected to correction and fusion by the spinal deformity correction and fusion system.

### Brief Description of Drawings

Fig. 1 is a perspective view showing a corrective appliance according to a first embodiment of the present invention and showing a state where a screw member is clamped using the corrective appliance;
Fig. 2 is a perspective view of a screw member of a spinal deformity correction and fusion system to which the corrective appliance according to the first embodiment of the present invention is applied;
Fig. 3 is a perspective view of a hook member of the spinal deformity correction and fusion system to which the corrective appliance according to the first embodiment of the present invention is applied;
Fig. 4 is a perspective view of a clamp member used in the corrective appliance according to the first embodiment of the present invention;
Fig. 5 is a perspective view of the clamp member used in the corrective appliance according to the first embodiment of the present invention viewed from a different direction from Fig. 4;
Fig. 6 is a perspective view showing a first gear, a second gear, a rotary member, etc. used in the clamp member of the corrective appliance according to the first embodiment of the present invention;
Fig. 7 is a view showing stages of correction and fusion of spinal deformity performed by the spinal deformity correction and fusion system using the corrective appliance according to the first embodiment of the present invention;
Fig. 8 is a view showing stages subsequent to Fig. 7;
Fig. 9 is a perspective view showing a corrective appliance according to a second embodiment of the present invention and showing a state where a screw member is clamped using the corrective appliance;
Fig. 10 is a perspective view of a clamp member used in the corrective appliance according to the second embodiment of the present invention;
Fig. 11 is a perspective view of a main body unit forming the clamp member of the corrective appliance according to the second embodiment of the present invention;
Fig. 12 is a perspective view of the main body unit forming the clamp member of the corrective appliance according to the second embodiment of the present invention viewed from a different direction from Fig. 11;
Fig. 13 is a perspective view of a secondary body unit forming the clamp member of the corrective appliance according to the second embodiment of the present invention; and
Fig. 14 is a perspective view of a wedge slide member, an operative screw member, and a regulating plate member forming the clamp member of the corrective appliance according to the second embodiment of the present invention.

### Description of Embodiments

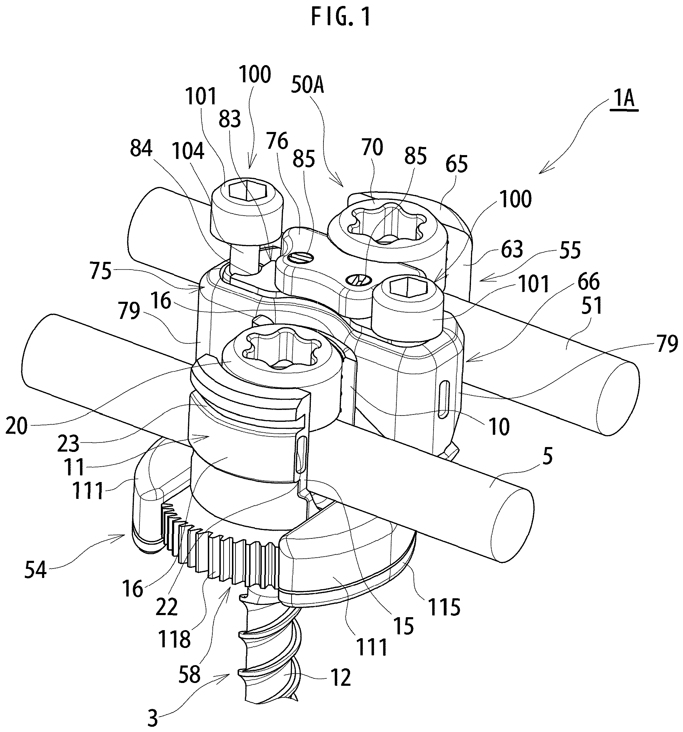

Embodiments for carrying out the present invention will be described below in detail on the basis of Figs. 1 to 14.

A corrective appliance 1A according to a first embodiment of the present invention will be described first in detail on the basis of Figs. 1 to 8.

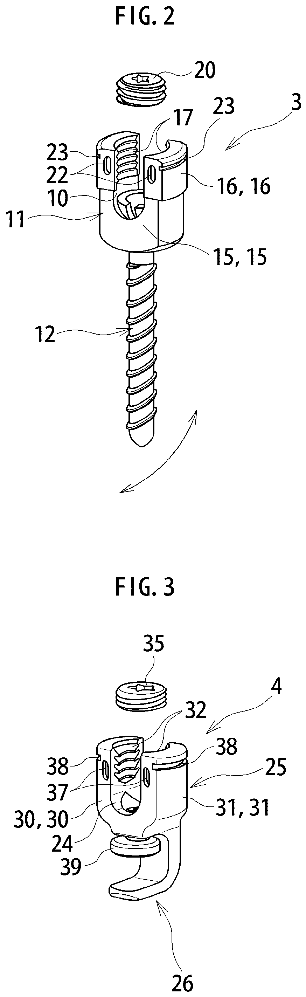

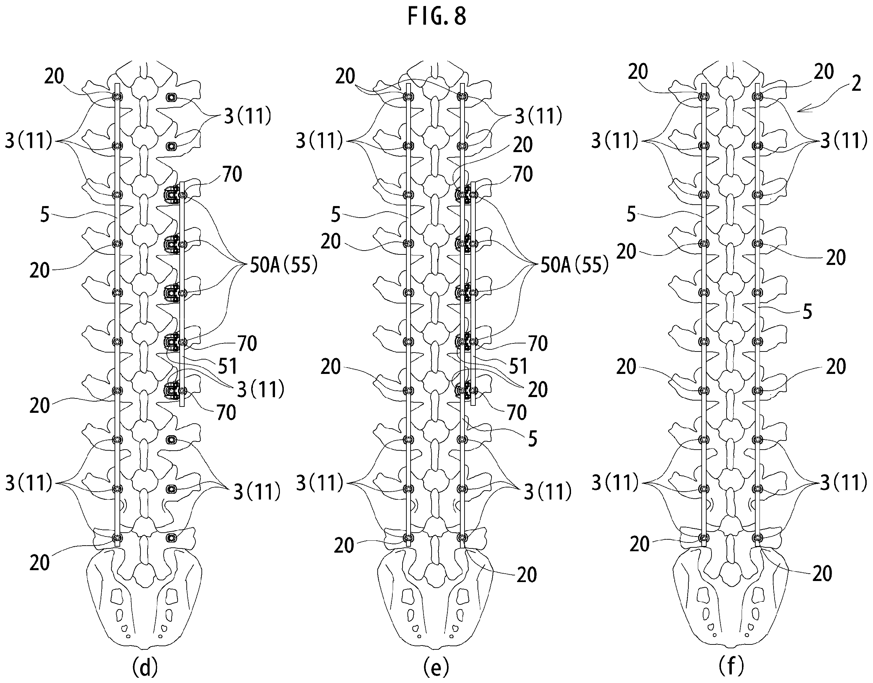

The corrective appliance 1A according to the first embodiment of the present invention is to assist in correction and fusion when spinal deformity is subjected to the correction and fusion by a spinal deformity correction and fusion system 2 (see Fig. 8(f)). The spinal deformity correction and fusion system 2 briefly includes: a plurality of screw members 3 (see Figs. 1 and 2) each to be screwed into a vertebral body through a pedicle of each vertebra of a spine; a plurality of hook members 4 (see Fig. 3) each to be hooked on a pedicle, a vertebral arch, or a transverse process, for example, of each vertebra; and a rod member 5 (see Fig. 1) to be coupled to a groove 10 of each screw member 3 and to a groove 24 of each hook member 4 and extending along an axis direction of a spine. These screw members 3 and hook members 4 correspond to a vertebra fixing tool. The spinal deformity correction and fusion system 2 further includes another constituting member such as a connector member (not shown in the drawings) provided to bridge the rod members 5, 5 in a pair. A plurality of the screw members 3 and the rod members 5, 5 in a pair of the spinal deformity correction and fusion system 2 are shown in Fig. 8(f).

The screw member 3, the hook member 4, and the rod member 5 are made of a material of excellent biocompatibility such as titanium alloy. As shown in Fig. 1, the rod member 5 is formed into a circular shape in a section. The length of the rod member 5 is set appropriately in response to a degree of spinal deformity of a patient. As shown in Figs. 1 and 2, the screw member 3 is to be screwed into a vertebral body through a pedicle of each vertebra from the back of a spine. The screw member 3 is generally called a pedicle screw. The screw member 3 includes a rod receiving section 11 (head) with a groove 10 to receive the rod member 5, and a screw portion 12 coupled to the rod receiving section 11 and to be screwed into a vertebral body through a pedicle of a vertebra.

The rod receiving section 11 is formed into a block-like shape in a plan view with planar portions 15, 15 in a pair and arc-like portions 16, 16 in a pair. The rod receiving section 11 is provided with the U-shape groove 10 opened on the opposite side of the screw portion 12 and extending in an axis direction of the rod member 5. The groove 10 is formed in such a manner as to penetrate the planar portions 15, 15 in a pair. The groove 10 is configured to receive the rod member 5. The rod receiving section 11 has walls with inner wall surfaces facing each other across the groove 10 at which corresponding female threads 17, 17 are formed. A set screw 20 is screwed into the female threads 17, 17. The planar portions 15, 15 of the rod receiving section 11 are provided with concaved sections 22, 22 in a pair formed at positions close to the respective tops of the planar portions 15, 15. The arc-like portions 16, 16 of the rod receiving section 11 have outer peripheral surfaces where engagement grooves 23, 23 extending in a peripheral direction are formed at positions close to the respective tops of the arc-like portions 16, 16. The screw portion 12 is coupled to the rod receiving section 11 in such a manner as to be swingable relative to the rod receiving section 11 in a direction in which the groove 10 extends. A range of the swinging motion of the screw portion 12 relative to the rod receiving section 11 is within 25° from the center to each side (a total swinging range of about 50°).

As shown in Fig. 3, the hook member 4 is to be engaged with a vertebra by being hooked on a pedicle, a vertebral arch, or a transverse process, for example, of the vertebra from the back of a spine. The hook member 4 includes a rod receiving section 25 (head) with a groove 24 to receive the rod member 5, and a hook portion 26 integrally connected to the rod receiving section 25. The rod receiving section 25 is formed into a block-like shape in a plan view with planar portions 30, 30 in a pair and arc-like portions 31, 31 in a pair. The rod receiving section 25 is provided with the U-shape groove 24 opened on the opposite side of the hook portion 26 and extending in the axis direction of the rod member 5. The groove 24 is formed in such a manner as to penetrate the planar portions 30, 30 in a pair. The U-shape groove 24 is configured to receive the rod member 5.

The rod receiving section 25 has walls with inner wall surfaces facing each other across the groove 24 at which corresponding female threads 32, 32 are formed. A set screw 35 is screwed into the female threads 32, 32. The planar portions 30 of the rod receiving section 25 are provided with concaved sections 37, 37 in a pair formed at positions close to the respective tops of the planar portions 30. The arc-like portions 31, 31 of the rod receiving section 25 have outer peripheral surfaces where engagement grooves 38, 38 extending in a peripheral direction are formed at positions close to the respective tops of the arc-like portions 31, 31. In some cases, the hook member 4 includes a sandwiching part 39 arranged in such a manner as to face the hook portion 26 and to be movable in an axis direction. The sandwiching part 39 and the hook portion 26 can be used for firmly sandwiching a transverse process, etc.

A corrective appliance 1A according to the first embodiment of the present invention will be described next in detail on the basis of Figs. 1 and 4 to 6. For the convenience of description, the following explanation is given on the assumption that, in Figs. 1 and 4 to 6, the screw portion 12 of the screw member 3 shown in Fig. 1 is on a lower side and the rod receiving section 11 of the screw member 3 is on an upper side (on the opposite side of the screw portion 12). The corrective appliance 1A according to the first embodiment of the present invention functions as a surgical instrument to be taken out of a body after spinal deformity is subjected to correction and fusion by the spinal deformity correction and fusion system 2 (see Fig. 8(f)). As shown in Fig. 1, the corrective appliance 1A according to the first embodiment includes a clamp member 50A to be attached detachably to the rod receiving section 11 (head) of the screw member 3 and to be arranged close to the rod receiving section 11 of the screw member 3, and an auxiliary rod member 51 as a corrective member to be attached detachably to the clamp member 50A and to be used for correcting spinal deformity.

As shown in Fig. 1, in the present embodiment, the clamp member 50A of the corrective appliance 1A according to the first embodiment is attached to the rod receiving section 11 of the screw member 3. The clamp member 50A is also attachable to and detachable from the rod receiving section 25 of the hook member 4 shown in Fig. 3. The auxiliary rod member 51 is formed into a circular shape in a section. The auxiliary rod member 51 has an outer diameter substantially the same as that of the rod member 5 of the spinal deformity correction and fusion system 2.

As shown in Figs. 1 and 4 to 6, the clamp member 50A used in the corrective appliance 1A according to the first embodiment includes: a screw support 54 with supporting pieces 111, 111 in a pair spaced apart from each other to support the rod receiving section 11 of the screw member 3; a rod receiving section 55 with a U-shape groove 63 with an opened upper surface; a first gear 56 arranged in the rod receiving section 55, a second gear 57 in meshing engagement with the first gear 56 extending from the interior of the screw support 54 to the interior of the rod receiving section 55; and a rotary member 58 in meshing engagement with the second gear 57 and rotatably supported in such a manner as to bridge respective tips of the supporting pieces 111, 111 in a pair of the screw support 54. The supporting pieces 111, 111 in a pair of the screw support 54 and the rotary member 58 correspond to a ring-like member.

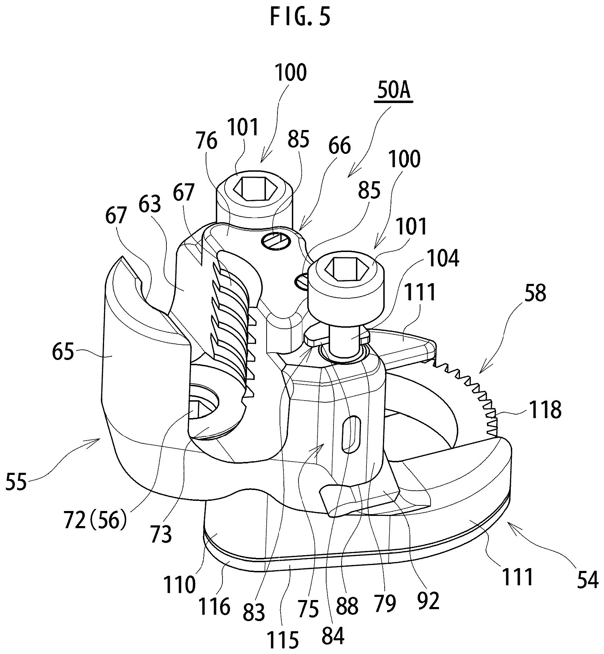

The screw support 54 and the rod receiving section 55 are each composed of a single part. As understood from Fig. 5, the screw support 54 and the rod receiving section 55 are connected integrally to each other in such a manner as to overlap each other partially, and are arranged side by side in a top-bottom direction in a plan view. The rod receiving section 55 has the U-shape groove 63 with the opened upper surface. The groove 63 is formed in a direction in which the rod member 5 extends. One of walls defined across the groove 63 of the rod receiving section 55 is formed into an arc-like wall 65 having an arc-like outer peripheral surface. The other of the walls defined across the groove 63 is formed into a block-like wall 66 having a shape like a block as a whole. A surface of the arc-like wall 65 and that of the block-like wall 66 facing each other are provided with corresponding female threads 67, 67. A set screw 70 (see Fig. 1) is screwed into the female threads 67, 67. The set screw 70 corresponds to a fixing tool.

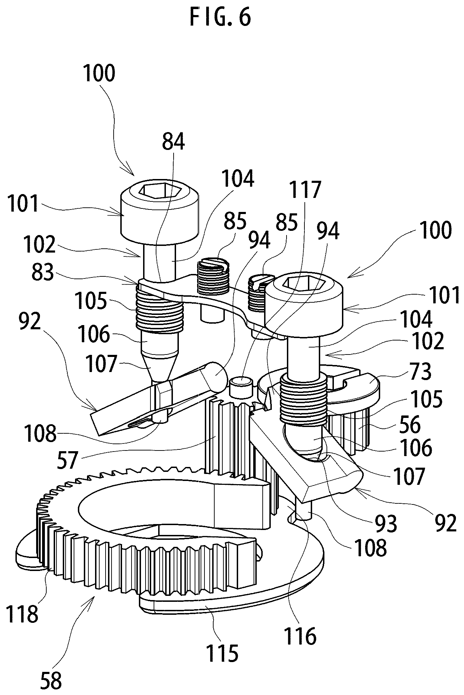

While not shown in the drawings, the bottom of the groove 63 of the rod receiving section 55 is provided with a circular concaved section opened at the bottom, and a through hole formed concentrically with the bottom of the circular concaved section. The first gear 56 (see Fig. 6) is rotatably supported in the circular concaved section. As understood from Fig. 5, a polygonal hole 72 penetrating the first gear 56 is formed at the center of the first gear 56 in a radial direction. The first gear 56 can be rotated by fitting a dedicated surgical instrument in the polygonal hole 72 and operating the fitted surgical instrument. A C-ring 73 is arranged on the first gear 56. The C-ring 73 projects slightly from the bottom surface of the groove 63 of the rod receiving section 55.

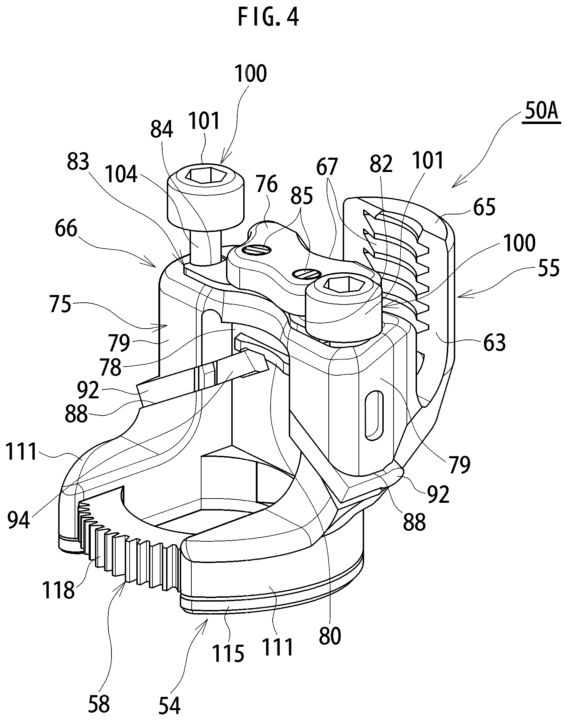

The block-like wall 66 is composed of a block body 75 and a supporting wall 76 projecting upward from the block body 75. The above-described female threads 67 are formed in a range from the supporting wall 76 to the block body 75. As understood from Fig. 4, a surface of the block body 75 close to the screw support 54 is provided with a supporting cavity 78 for supporting one of the arc-like portions 16 of the rod receiving section 11 of the screw member 3 (see Fig. 2). The supporting cavity 78 has a bottom surface where an engagement convex 80 is formed that is to be engaged with the engagement groove 23 (see Fig. 2) provided at the outer peripheral surface of the arc-like portion 16 of the rod receiving section 11 of the screw member 3. Poles 79, 79 in a pair are formed across the supporting cavity 78 in such a manner as to extend in a direction in which the groove 63 extends, and the poles 79 are provided with female screw holes (not shown in the drawings) with opened upper surfaces. A fixing screw member 100 described later is threadedly engaged with each of the female screw holes. The supporting wall 76 having a substantially trapezoidal shape in a plan view is provided between the female screw holes of the block body 75 in such a manner as to project upward from the block body 75.

A tiny gap 82 (see Fig. 4) is provided between the block body 75 and the supporting wall 76 and close to the screw support 54. A plate-like stopper member 83 is arranged in the gap 82. The stopper member 83 is fixed in the gap 82 using two screw members 85, 85. The stopper member 83 is formed into a shape elongated in the direction in which the groove 63 extends in a plan view. The stopper member 83 has opposite ends as viewed in its lengthwise direction where concaved stoppers 84, 84 are provided each arranged in such a manner as to block a tiny area around the female screw hole of the block body 75. By doing so, even if the fixing screw member 100 described later is rotated excessively in a direction of being pulled out, male threads 105 of the fixing screw member 100 interfere with the concaved stopper 84 of the stopper member 83, thereby preventing the fixing screw member 100 from coming off the corresponding female screw hole of the block body 75.

As shown in Figs. 4 and 5, the poles 79, 79 of the block body 75 are provided with corresponding supporting spaces 88, 88 rectangular in sections extending upward diagonally from outside toward the supporting cavity 78 in such a manner as to intersect the corresponding female screw holes and to penetrate the corresponding poles 79, 79. The supporting space 88 is formed in such a manner as to open the pole 79 on a side close to the screw support 54. By referring further to Fig. 6, a stopper pawl section 92 is supported in each of the supporting spaces 88 in such a manner as to be movable back and forth. The stopper pawl section 92 has a plate-like shape and is formed into a substantially rectangular shape in a plan view. The stopper pawl section 92 is provided with a long hole 93 extending in a lengthwise direction in such a manner as to penetrate the stopper pawl section 92. The fixing screw member 100 described latter is passed through the long hole 93. The stopper pawl section 92 has a tip where a pawl 94 is formed at an end thereof as viewed in a width direction in such a manner as to project in a lengthwise direction. The stopper pawl section 92 is configured to allow the pawl 94 at the tip of the stopper pawl section 92 to go into and out of the supporting cavity 78 of the block body 75, as will be described later.

As shown in Fig. 6, the fixing screw member 100 is composed of a hexagonal hole head 101, and a shaft 102 with male threads 105. The shaft 102 is composed of a small-diameter shaft section 104, the male threads 105, a large-diameter shaft section 106, a tapered shaft section 107, and a minimum-diameter shaft section 108 arranged in this order as viewed from the head 101 toward a tip. The small-diameter shaft section 104 has an outer diameter less than the outer diameter of the male threads 105. The large-diameter shaft section 106 has an outer diameter less than the outer diameter of the male threads 105 and greater than the outer diameter of the small-diameter shaft section 104. The tapered shaft section 107 is formed in such a manner that the outer diameter thereof is reduced gradually from the large-diameter shaft section 106 toward the minimum-diameter shaft section 108.

As shown in Figs. 4 to 6, the male threads 105 of the fixing screw member 100 are screwed into the female screw hole of the pole 79 of the block body 75 and the tapered shaft section 107 of the fixing screw member 100 is passed through the interior of the long hole 93 of the stopper pawl section 92 arranged in the supporting space 88 of each pole 79. As the fixing screw member 100 is screwed in and is moved in the axis direction, the fixing screw member 100 is moved while the tapered shaft section 107 of the fixing screw member 100 is in contact with the inner wall surface of the long hole 93 of the stopper pawl section 92. This allows the stopper pawl section 92 to go into and out of the supporting cavity 78 of the block body 75.

As understood from Fig. 5, the screw support 54 includes: a gear housing 110 connected to the rod receiving section 55 in such a manner as to overlap the rod receiving section 55 in the top-bottom direction, and the supporting pieces 111, 111 in a pair provided integrally with the gear housing 110 and extending like branches from the gear housing 110, and each provided integrally with and extending from a lower end portion of each of the poles 79, 79 of the block body 75 of the rod receiving section 55. The gear housing 110 has an opened lower section and the second gear 57 (see Fig. 6) is housed in the gear housing 110. Each of the supporting pieces 111, 111 in a pair is provided with a housing cavity (not shown in the drawings) having an opened lower section. The rotary member 58 is arranged in the housing cavities in the supporting pieces 111, 111 in a pair in such a manner as to cross the gear housing 110. A substantially C-shaped cover member 115 is arranged at the screw support 54 in such a manner as to cover the second gear 57 in the gear housing 110 and to cover the rotary member 58 in the supporting pieces 111, 111 in a pair from below.

By referring to Fig. 6, the cover member 115 is provided with a projection 116 projecting outward in a radial direction toward the opposite side of the opened part thereof in the radial direction. The second gear 57 is supported rotatably on a support pin 117 standing from the projection 116. The second gear 57 is arranged to cover an area from the gear housing 110 of the screw support 54 to a position adjacent to the first gear 56 in the bottom of the rod receiving section 55, and is in meshing engagement with the first gear 56. The second gear 57 is also in meshing engagement with a gear 118 of the rotary member 58. A distance between the supporting pieces 111, 111 in a pair is set to a distance that allows the planar portions 15, 15 in a pair at the rod receiving section 11 of the screw member 3 to come into abutting contact with the supporting pieces 111, 111 in a pair and to be fitted therebetween.

The rotary member 58 is formed into a C-shape. The rotary member 58 extends in the housing cavities in the supporting pieces 111, 111 in a pair in such a manner as to cross the gear housing 110, and is rotatably supported in such a manner as to bridge the tips of the supporting pieces 111, 111 in a pair. The rotary member 58 has an outer peripheral surface where the gear 118 is formed. The gear 118 is in meshing engagement with the second gear 57. The inner diameter of the rotary member 58 is substantially equal to an outer diameter defined by the arc-like portions 16, 16 in a pair of the rod receiving section 11 of the screw member 3 (see Fig. 2). Rotating the first gear 56 using a dedicated surgical instrument allows the second gear 57 and the rotary member 58 to rotate. The resultant rotation of the rotary member 58 makes it possible to open and close the supporting pieces 111, 111 in a pair of the screw support 54 relative to each other.

By referring to Figs. 1 and 4 to 6, for attachment of the clamp member 50A to the rod receiving section 11 of the screw member 3, a dedicated surgical instrument (not shown in the drawings) is fitted in the polygonal hole 72 of the first gear 56 arranged in the bottom of the groove 63 of the rod receiving section 55 and is then rotated to one direction, thereby rotating the first gear 56, the second gear 57, and the rotary member 58 to open the supporting pieces 111, 111 in a pair of the screw support 54 relative to each other. Next, while one of the arc-like portions 16 of the rod receiving section 11 of the screw member 3 is located in the supporting cavity 78 provided at the block body 75 of the block-like wall 66 at the rod receiving section 55 of the clamp member 50A, the supporting pieces 111, 111 in a pair of the screw support 54 of the clamp member 50A are brought into abutting contact with the planar portions 15, 15 in a pair of the rod receiving section 11 of the screw member 3 at positions below the groove 10. Locating the one arc-like portion 16 of the rod receiving section 11 of the screw member 3 in the supporting cavity 78 at the rod receiving section 55 of the clamp member 50A engages the engagement groove 23 (see Fig. 2) provided at the outer peripheral surface of the one arc-like portion 16 of the rod receiving section 11 of the screw member 3 with the engagement convex 80 provided at the supporting cavity 78 of the rod receiving section 55 of the clamp member 50A (see Fig. 4). By doing so, the movement of the clamp member 50A in the top-bottom direction relative to the screw member 3 is regulated.

Next, the dedicated surgical instrument (not shown in the drawings) is fitted again in the polygonal hole 72 of the first gear 56 arranged in the bottom of the groove 63 of the rod receiving section 55 and is then rotated to the opposite direction, thereby rotating the first gear 56, the second gear 57, and the rotary member 58 to close the supporting pieces 111, 111 in a pair of the screw support 54 relative to each other using the rotary member 58. As a result, the inner peripheral surface of the rotary member 58 is brought into abutting contact with the outer peripheral surface of the other arc-like portion 16 at the rod receiving section 11 of the screw member 3. By doing so, while the rod receiving section 11 of the screw member 3 is held using the rotary member 58, the groove 10 of the screw member 3 (rod receiving section 11) is exposed to the outside.

Next, a dedicated surgical instrument (not shown in the drawings) is fitted in the hexagonal hole head 101 of each fixing screw member 100 and the surgical instrument is rotated to one direction. By doing so, each fixing screw member 100 is screwed in to cause each fixing screw member 100 to move forward in the axis direction. At this time, each fixing screw member 100 moves forward while the tapered shaft section 107 of each fixing screw member 100 contacts the inner wall surface of the long hole 93 of each stopper pawl section 92, thereby causing each stopper pawl section 92 to move forward toward the interior of the supporting cavity 78 of the block body 75. As a result, the pawl 94 of each stopper pawl section 92 presses the concaved section 22 at each planar portion 15 of the rod receiving section 11 of the screw member 3. By doing so, the rod receiving section 11 of the screw member 3 is sandwiched and fixed firmly from the sides of the planar portions 15, 15 in a pair with the stopper pawl sections 92, 92 in a pair. In this way, by attaching the clamp member 50A to the rod receiving section 11 of the screw member 3, the clamp member 50A is located close to the rod receiving section 11 of the screw member 3.

For detaching the clamp member 50A from the rod receiving section 11 of the screw member 3, like in the case of attaching the clamp member 50A, a dedicated surgical instrument (not shown in the drawings) is fitted in the hexagonal hole head 101 of each fixing screw member 100 and the surgical instrument is rotated to the opposite direction, thereby causing each fixing screw member 100 to move backward in the axis direction. This causes the tapered shaft section 107 of each fixing screw member 100 to move backward to cause each stopper pawl section 92 to move slidably under its own weight inside the supporting space 88. As a result, the pawl 94 of each stopper pawl section 92 is separated from the concaved section 22 at each planar portion 15 of the rod receiving section 11 of the screw member 3, thereby releasing the rod receiving section 11 of the screw member 3 from the sandwiching action exerted by each stopper pawl section 92.

Furthermore, a dedicated surgical instrument (not shown in the drawings) is fitted in the polygonal hole 72 of the first gear 56 arranged in the bottom of the groove 63 of the rod receiving section 55 and is rotated to one direction, thereby rotating the first gear 56, the second gear 57, and the rotary member 58 to open the supporting pieces 111, 111 in a pair of the screw support 54 relative to each other. In this state, the clamp member 50A becomes detachable from the rod receiving section 11 of the screw member 3.

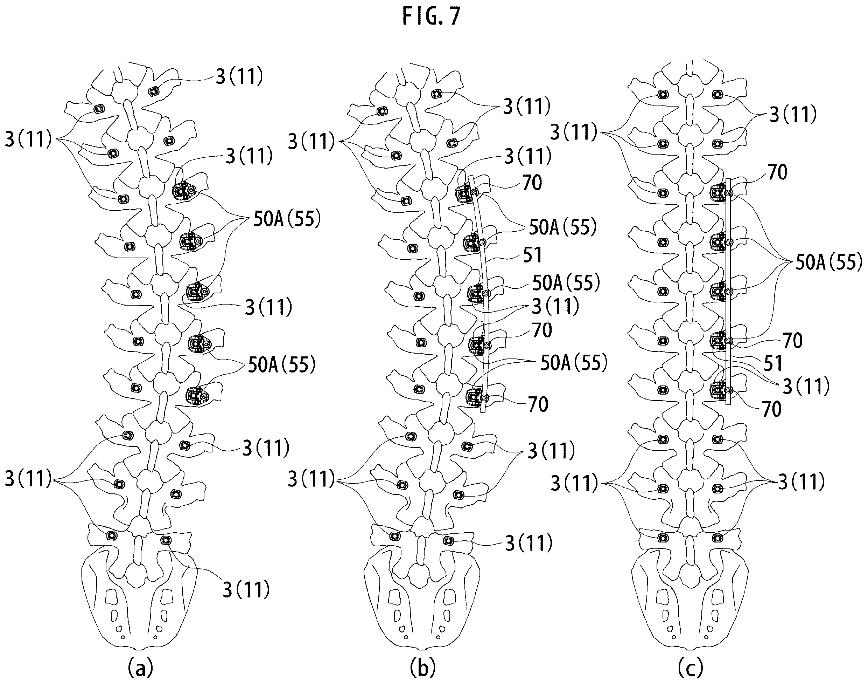

The following describes a method (not claimed) of assisting in correction and fusion using the corrective appliance 1A according to the first embodiment when spinal deformity is subjected to the correction and fusion by the spinal deformity correction and fusion system 2 on the basis of Figs. 7 and 8 and by also referring to Figs. 1 and 4, if appropriate. While the spinal deformity correction and fusion system 2 in Figs. 7 and 8 simply performs correction and fusion of the spinal deformity by using only a plurality of the screw members 3 and the rod members 5 in a pair, this is intended to facilitate understanding of the method of assisting in the correction and fusion using the corrective appliance 1A according to the first embodiment. **In** some actual cases, a member such as the hook member 4 or a connector member (not shown in the drawings) is used as well as the screw member 3, or another rod member is used in addition to the rod members 5, 5 in a pair, if necessary.

As shown in Fig, 7(a), in response to spinal deformity such as scoliosis, the screw member 3 is first screwed into a vertebral body through a pedicle of each of vertebrae (ten vertebrae in the drawing, for example) in a range of correction and fusion of a spine from the back of the spine. Next, in response to a case such as a single curve like the one shown in Fig. 7(a), for example, the clamp member 50A is attached to the rod receiving section 11 of the screw member 3 screwed into each of vertebrae that may be five vertebrae within a range from the apex to the vicinity of the apex of the curve, for example. In this method (not claimed) the clamp member 50A is attached in such a manner that the rod receiving section 55 of this clamp member 50A is arranged external to the rod receiving section 11 of the screw member 3 as viewed in a crosswise direction of a patient. The explanation of the method of attaching the clamp member 50A to the rod receiving section 11 of the screw member 3 is omitted here as it has already been given above.

Next, one auxiliary rod member 51 is prepared and the auxiliary rod member 51 is curved using a dedicated surgical instrument (not shown in the drawings) along a curve of the scoliosis of the spine. Then, as shown in Fig. 7(b), the auxiliary rod member 51 is engaged with the groove 63 of the rod receiving section 55 of each clamp member 50A. Next, the set screw 70 is screwed into the female threads 67, 67 of the rod receiving section 55 of each clamp member 50A using a dedicated surgical instrument (not shown in the drawings) to temporarily tighten the auxiliary rod member 51. At this time, when the auxiliary rod member 51 is pressed with the set screw 70 against the bottom surface of the groove 63 of the rod receiving section 55 of each clamp member 50A, the C-ring 73 on the first gear 56 presses the first gear 56 against the bottom of the circular concaved section of the rod receiving section 55. By doing so, the rotation of the first gear 56 is regulated, so that the rod receiving section 11 (head) of the screw member 3 is continuously held using the rotary member 58.

Next, in the state of Fig. 7(b), operation is performed using a dedicated surgical instrument (not shown in the drawings) to grasp and rotate the auxiliary rod member 51 or to move the clamp members 50A in a direction of getting closer to or farther from each other, thereby correcting the spinal deformity including twisting. Next, as shown in Fig. 7(c), the set screw 70 of the corrective appliance 1A according to the first embodiment is fully tightened to fix the auxiliary rod member 51 firmly to the groove 62 of the rod receiving section 55 of each clamp member 50A. In this way, it becomes possible to correct the spinal deformity and to retain the corrected state using the corrective appliance 1A according to the first embodiment. Next, as shown in Fig. 8(d), the rod member 5 is engaged with the grooves 10 of the rod receiving sections 11 of all the screw members 3 (ten screw members 3) on the left side without the attached clamp member 50A. Then, using a dedicated surgical instrument (not shown in the drawings), the set screw 20 is screwed into the female threads 17, 17 of the rod receiving section 11 of each screw member 3 (see Fig. 2) and fully tightened, thereby fixing the rod member 5 firmly to the groove 10 of the rod receiving section 11 of each screw member 3. At this time, the rod member 5 may be curved slightly in such a manner as to conform to (physiological) normal backward curvature and forward curvature of the patient, if necessary, and the rod member 5 in this state may be engaged with the grooves 10 of the rod receiving sections 11 of all the screw members 3 (ten screw members 3).

Next, as shown in Fig. 8(e), the rod member 5 is engaged with the grooves 10 of the rod receiving sections 11 of all the screw members 3 (ten screw members 3) on the right side on which the clamp members 50A are attached. Furthermore, the set screw 20 is screwed into the female threads 17, 17 of the rod receiving section 11 of each screw member 3 (see Fig. 2) and fully tightened, thereby fixing the rod member 5 firmly to the groove 10 of the rod receiving section 11 of each screw member 3.

Next, as shown in Fig. 8(f), the set screw 70 is detached from the rod receiving section 55 of each clamp member 50A and the auxiliary rod member 51 is detached from each clamp member 50A. Then, each clamp member 50A is detached from the rod receiving section 11 of each screw member 3. By doing so, the correction and fusion of the spinal deformity by the spinal deformity correction and fusion system 2 (a plurality of the screw members 3 and the rod members 5 in a pair) is completed. The explanation of the method (not claimed) of detaching the clamp member 50A from the rod receiving section 11 of the screw member 3 is omitted here as it has already been given above.

In the method (not claimed) shown in Figs. 7 and 8, the clamp member 50A is attached in such a manner that the rod receiving section 55 of this clamp member 50A is arranged external to the rod receiving section 11 of the screw member 3 as viewed in the crosswise direction of the patient. Alternatively, the clamp member 50A may be attached to the rod receiving section 11 of the screw member 3 in such a manner that the rod receiving section 55 of this clamp member 50A is arranged internal to the rod receiving section 11 of the screw member 3 as viewed in the crosswise direction of the patient. In the method shown in Figs. 7 and 8, the clamp member 50A is attached to the rod receiving section 11 of each screw member 3 on the right side as viewed in the crosswise direction of the patient. Alternatively, the clamp member 50A may be attached to the rod receiving section 11 of each screw member 3 on the left side as viewed in the crosswise direction. Still alternatively, the clamp member 50A may be attached to the rod receiving section 11 of each screw member 3 on the both sides as viewed in the crosswise direction.

As described above, in addition to the method (not claimed) shown in Figs. 7 and 8, correction and fusion of spinal deformity by the spinal deformity correction and fusion system 2 may be assisted in response to an intention by an operator by attaching the clamp member 50A to the rod receiving section 11 of the selected screw member 3 and performing operation for correction using the attached clamp member 50A and the auxiliary rod member 51. In summary, the corrective appliance 1A according to the first embodiment is applicable not only to the method shown in Figs. 7 and 8 but to a method (not claimed) different from the method shown in Figs. 7 and 8 responsive to an intention by an operator.

As described above, the corrective appliance 1A according to the first embodiment of the present invention includes: the clamp member 50A to be attached detachably, for example, to the rod receiving section 11 of the screw member 3 of the spinal deformity correction and fusion system 2 and to be arranged close to the rod receiving section 11 of the screw member 3; and the auxiliary rod member 51 to be attached detachably to the rod receiving section 55 of the clamp member 50A and to be used for correcting spinal deformity.

In this configuration, the clamp member 50A is attached to each of a plurality of the screw members 3, and the auxiliary rod member 51 is attached further to the groove 63 of the rod receiving section 55 of each clamp member 50A. Next, the auxiliary rod member 51 is operated, making it possible to correct the spinal deformity properly through each clamp member 50A and each screw member 3 and to retain the corrected state. Then, the rod member 5 is coupled to the groove 10 of the rod receiving section 11 of each screw member 3 to fix a spine in the corrected state. Next, the auxiliary rod member 51 and each clamp member 50A are detached from each screw member 3 and then taken out of a body.

During the correction and fusion of the spinal deformity in this way by the spinal deformity correction and fusion system 2, unlike in the conventional case, it is not necessary to engage the rod member 5 of the spinal deformity correction and fusion system 2 with each screw member 3 or each hook member 4 fixed to each vertebra under scoliosis deformity including twisting, or to perform operation of giving a curve to the rod member 5 of the spinal deformity correction and fusion system 2 along the scoliosis deformity or operation of firmly sandwiching the rod member 5 and rotating the rod member 5 using a dedicated surgical instrument such as a pair of pliers. As a result, it becomes possible to limit damage on the rod member 5 as a self-contained member. Furthermore, using the corrective appliance 1A according to the first embodiment allows the rod member 5 of the spinal deformity correction and fusion system 2 to be easily engaged with (coupled to) the groove 10 (24) of the rod receiving section 11 (25) of each screw member 3 (hook member 4). As a result, correction of the spinal deformity by the spinal deformity correction and fusion system 2 becomes easier to facilitate implementation of a surgery itself, thereby contributing to reduction in a duration of the surgery.

The clamp member 50A used in the corrective appliance 1A according to the first embodiment of the present invention includes, as a ring-like member to support the rod receiving section 11 of the screw member 3 in such a manner as to surround an outer periphery of the rod receiving section 11, the supporting pieces 111, 111 in a pair of the screw support 54, and the rotary member 58 rotatably supported in such a manner as to open and close respective tips of the supporting pieces 111, 111 in a pair relative to each other. This allows the groove 10 provided at the rod receiving section 11 of the screw member 3 and used for engagement with the rod member 5 to be exposed to the outside. As a result, while spinal deformity is corrected and the corrected state is retained using the clamp member 50A and the auxiliary rod member 51, the rod member 5 can be engaged easily with the groove 10 of each screw member 3.

The clamp member 50A used in the corrective appliance 1A according to the first embodiment of the present invention further includes the set screw 70 to be used for fixing the auxiliary rod member 51 to the groove 63 of the rod receiving section 55 of the clamp member 50A. By operating the auxiliary rod member 51 while the auxiliary rod member 51 is temporarily tightened to the groove 63 of each clamp member 50A using the set screw 70, it becomes possible to correct spinal deformity easily through each clamp member 50A. After the correction, the auxiliary rod member 51 is fixed firmly to the groove 63 of the rod receiving section 55 of each clamp member 50A using the set screw 70. By doing so, the spinal deformity can be retained in the corrected state using the clamp member 50A and the auxiliary rod member 51.

The clamp member 50A used in the corrective appliance 1A according to the first embodiment of the present invention further includes the stopper pawl sections 92, 92 in a pair for sandwiching the rod receiving section 11 of the screw member 3, in addition to the supporting pieces 111, 111 in a pair of the screw support 54 and the rotary member 58 rotatably supported in such a manner as to open and close respective tips of the supporting pieces 111, 111 in a pair relative to each other. The stopper pawl sections 92, 92 in a pair can be used for fixing the clamp member 50A to the screw member 3 more firmly. As a result, force of the correction applied from the clamp member 50A can be transmitted properly to a vertebra through the screw member 3, making it possible to correct spinal deformity properly.

Furthermore, the planar portion 15 of the screw member 3 is provided with the concaved section 22 for supporting the tip of the pawl 94 of the stopper pawl section 92 of the corrective appliance 1A according to the first embodiment of the present invention. When the clamp member 50A is attached to the screw member 3, the stopper pawl section 92 of the clamp member 50A is engaged with the concaved section 22 of the screw member 3. This makes it possible to minimize backlash of the clamp member 50A relative to the screw member 3 occurring during attachment of the clamp member 50A to the screw member 3.

The corrective appliance 1A according to the first embodiment uses the auxiliary rod member 51 as a corrective member to be engaged with the groove 63 of the rod receiving section 55 of the clamp member 50A. This corrective member may be a shaft member, for example, to be attached detachably to the clamp member 50A and to extend from the clamp member 50A toward the outside of a body. If such a shaft member is used, spinal deformity can be corrected properly by causing an operator to grasp and operate each shaft member projecting to the outside of the body or to operate the shaft member using a surgical instrument. A member such as that for retention of correction for retaining a corrected state may be mounted on a tip of each shaft member.

While the corrective appliance 1A according to the first embodiment functions as a surgical instrument to be taken out of a body after spinal deformity is subjected to correction and fusion by the spinal deformity correction and fusion system 2, it can indwell as it is in the body. In this case, like the screw member 3, the hook member 4, the rod member 5, etc., of the spinal deformity correction and fusion system 2, the corrective appliance 1A according to the first embodiment is required to be made of a material of excellent biocompatibility such as titanium alloy. This allows improvement of the rigidity of the spinal deformity correction and fusion system 2 as a whole, making it possible to reduce the occurrence of breakage of the rod member 5, for example.

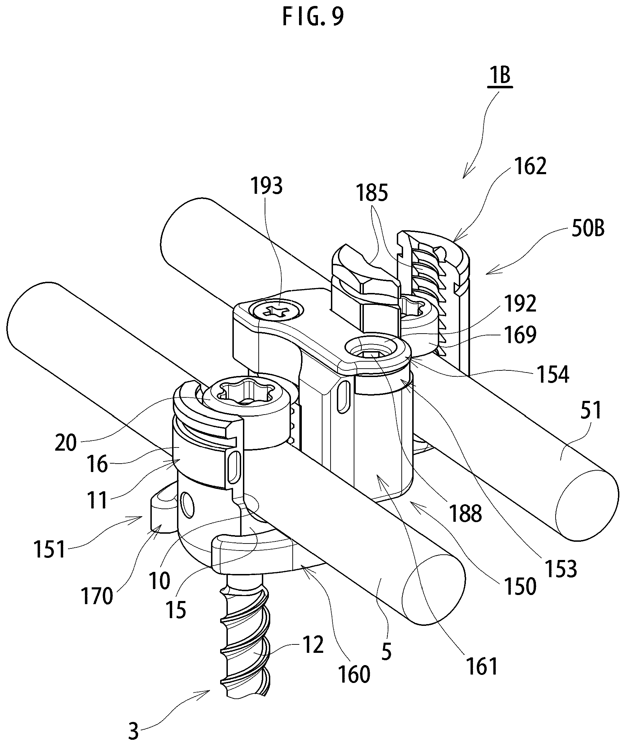

A corrective appliance 1B according to a second embodiment of the present invention will be described in detail on the basis of Figs. 9 to 14. In describing the corrective appliance 1B according to the second embodiment, only a difference from the corrective appliance 1A according to the first embodiment will be described. In the corrective appliance 1B of the second embodiment, the configuration of a clamp member 50B used in the corrective appliance 1B differs from that of the clamp member 50A used in the corrective appliance 1A according to the first embodiment. Thus, in the following, the configuration of the clamp member 50B will be described in detail.

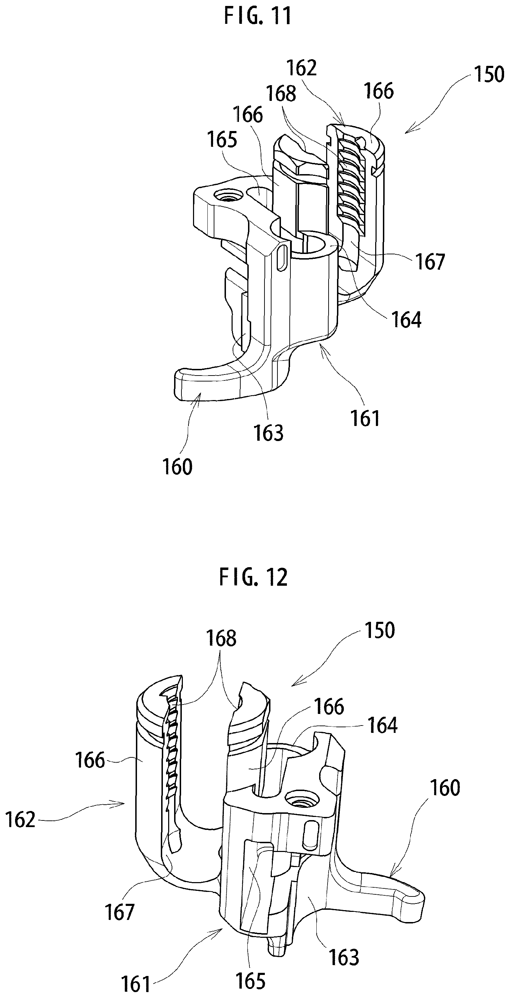

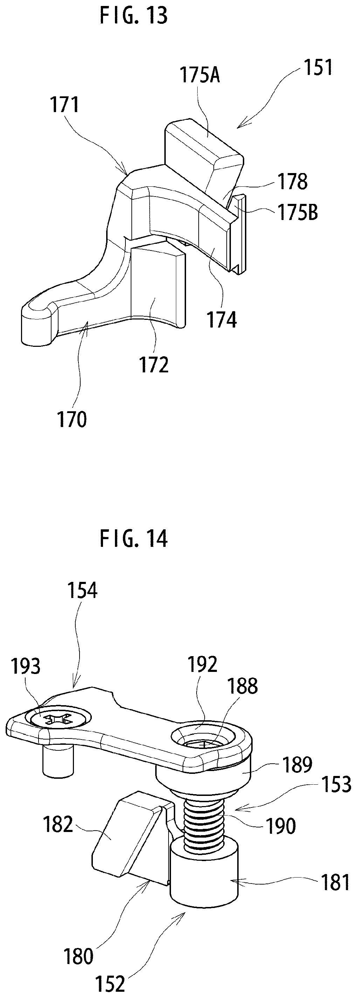

As shown in Figs. 9, 10, and 14, the clamp member 50B includes: supporting pieces 160, 170 in a pair movable closer to and farther from each other to support the rod receiving section 11 of the screw member 3; a rod receiving section 162 connected integrally to the one supporting piece 160 of the supporting pieces 160, 170 in a pair and having a U-shape groove 167 with an opened upper surface; and a wedge slide member 152 to move in the top-bottom direction to make the other supporting piece 170 of the supporting pieces 160, 170 in a pair movable closer to and farther from the one supporting piece 160. In other words, the clamp member 50B is formed by coupling and integrating a main body unit 150 shown in Figs. 11 and 12, a secondary body unit 151 shown in Fig. 13, the wedge slide member 152 shown in Fig. 14, an operative screw member 153 shown in Fig. 14, and a regulating plate member 154 shown in Fig. 14 each configured using a single part.

As shown in Figs. 11 and 12, the main body unit 150 is formed by connecting the one supporting piece 160, a main body unit section 161, and the rod receiving section 162 integrally to each other. The one supporting piece 160 extends from the lower end of the main body unit section 161 in a direction substantially orthogonal to a direction in which the groove 167 of the rod receiving section 162 extends. The one supporting piece 160 extends from the lower end of the main body unit section 161, which corresponds to one end side as viewed in the direction in which the groove 167 of the rod receiving section 162 extends. The main body unit section 161 has a front side (a side closer to the one supporting piece 160) where an arc-like screw abutting surface 163 is provided in such a manner as to be continuous with the one supporting piece 170. One of the arc-like portions 16 provided at the rod receiving section 11 of the screw member 3 shown in Fig. 9 is to abut on the screw abutting surface 163. The main body unit section 161 has a back side (a side on the opposite side of the one sporting piece 160) to which the rod receiving section 162 is integrally connected.

A housing hollow section 165 is formed in the main body unit section 161. The housing hollow section 165 has an opened upper surface having an arbitrary shape, is opened at the other end side as viewed in the direction in which the groove 167 of the rod receiving section 162 extends, and has an opened front side (a side closer to the one supporting piece 160) having a substantially rectangular shape as viewed in the direction in which the groove 167 of the rod receiving section 162 extends. A secondary body unit section 171 of the secondary body unit 151 and the wedge slide member 152 described later in detail are housed in the housing hollow section 165 while a wedge projection 182 provided at the wedge slide member 152 is engaged with an inclined space 178 provided at the secondary body unit section 171. A housing cavity 164 is formed at the upper surface of the main body unit section 161 and at one end of the main body unit section 161 closer to the one supporting piece 160.

The rod receiving section 162 has the U-shape groove 167 with the opened upper surface. The groove 167 is formed in the direction in which the rod member 5 extends (see Fig. 9). Walls 166, 166 facing each other across the groove 167 of the rod receiving section 162 have inner wall surfaces at which corresponding female threads 168, 168 are formed. A set screw 169 shown in Fig. 9 is screwed into the female threads 168, 168. The set screw 169 corresponds to a fixing tool for fixing the auxiliary rod member 51 to the groove 167 of the clamp member 50B.

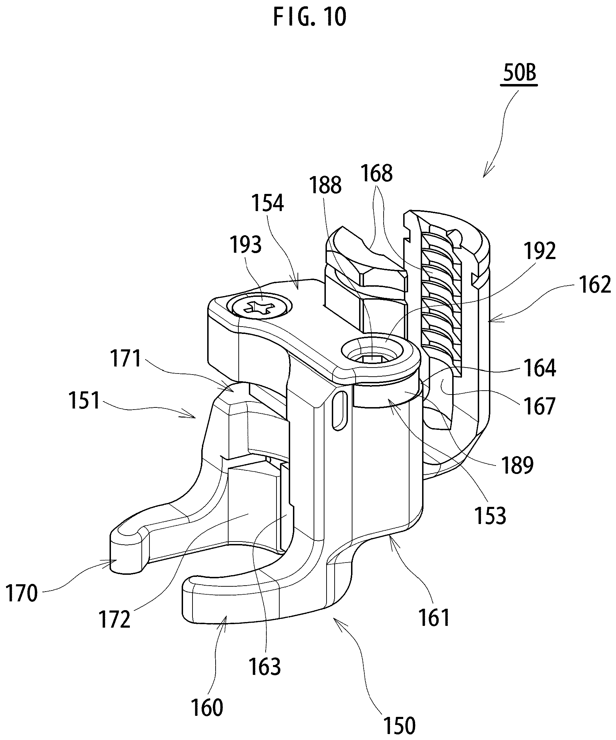

The rod receiving section 162, specifically, the wall 166, 166 thereof project more upward than the upper surface of the main body unit section 161 as shown in Fig. 11, and more upward than the regulating plate member 154 fixed in such a manner as to cover the upper surface of the main body unit section 161 as shown in Fig. 10. This allows a rod-like surgical instrument (not shown in the drawings), used for assisting in the operation of inserting the auxiliary rod member 51 (see Fig. 9) into the groove 167 of the rod receiving section 162, to be mounted at its tip on the projecting portions of the walls 166, 166. As shown in Fig. 11, the bottom surface of the groove 167 of the rod receiving section 162 is located above the one supporting piece 160. As shown in Fig. 13, the secondary body unit 151 is formed by connecting the other supporting piece 170 and the secondary body unit section 171 to each other integrally. The other supporting piece 170 extends from the lower end of the secondary body unit section 171 in a direction substantially orthogonal to the direction in which the groove 167 of the rod receiving section 162 extends. The other supporting piece 170 extends from the lower end of the secondary body unit section 171, which corresponds to the other end side as viewed in the direction in which the groove 167 of the rod receiving section 162 extends.

The secondary body unit section 171 includes: a plate-like body 174 having a plate-like shape of a predetermined width and extending in a standing position in the direction in which the groove 167 of the rod receiving section 162 extends; and a large inclination piece 175A and a small inclination piece 175B in a pair connected integrally to the back surface (a surface on the opposite side of the other supporting piece 170) of the plate-like body 174. An arc-like screw abutting surface 172 is provided spaced from and below the plate-like body 174 of the secondary body unit section 171 in such a manner as to be continuous with the other supporting piece 170. One of the arc-like portions 16 at the rod receiving section 11 of the screw member 3 shown in Fig. 9 is to abut on the screw abutting surface 172. The inclined space 178 of a predetermined width inclined downward toward the other supporting piece 170 (toward the other end) is formed between the large inclination piece 175A and the small inclination piece 175B in a pair. The thickness of the small inclination piece 175B on one end side is smaller than that of the large inclination piece 175A on the other end side. The wedge projection 182 provided at the wedge slide member 152 (see Fig. 14) is engaged with the inclined space 178.

As shown in Fig. 14, the wedge slide member 152 includes a plate-like section 180 and a cylindrical section 181 connected integrally to one end portion of the plate-like section 180. The wedge slide member 152 is housed in its entirety in the housing hollow section 165 at the main body unit section 161 of the main body unit 150. The plate-like section 180 is provided in a standing position, and has a front side where the wedge projection 182 projects at the other end on the opposite side of the cylindrical section 181. The wedge projection 182 is formed into a shape like a parallelogram in a front view having a predetermined width and inclined downward toward an attachment bolt 193 (toward the other end). The wedge projection 182 is engaged with the inclined space 178 at the secondary body unit 151 and has a width substantially equal to that of the inclined space 178. The cylindrical section 181 has an inner peripheral surface provided with female threads (not shown in the drawings). Male threads 190 of the operative screw member 153 are threadedly engaged with these female threads.

The operative screw member 153 is composed of a head 189 having an upper surface with a hexagonal hole 188, and the male threads 190 provided continuously in an axis direction from the head 189. The male threads 190 are threadedly engaged with the female threads provided at the inner peripheral surface of the cylindrical section 181 of the wedge slide member 152. The head 189 of the operative screw member 153 is housed in the housing cavity 164 provided at the upper surface of the main body unit section 161 of the main body unit 150. The regulating plate member 154 for regulating the movement of the operative screw member 153 in the top-bottom direction is arranged on the head 189 of the operative screw member 153. Specifically, as shown in Fig. 10, the head 189 of the operative screw member 153 is sandwiched between the housing cavity 164 of the main body unit 150 (main body unit section 161) and the regulating plate member 154 to support the operative screw member 153 rotatably while being unmovable in the top-bottom direction.

The regulating plate member 154 is provided with an opening 192 formed at a position corresponding to the head 189 of the operative screw member 153 so as to expose the hexagonal hole 188 at the head 189 of the operative screw member 153 to the outside. As shown in Fig. 10, the regulating plate member 154 is fixed to the main body unit 150 with the attachment bolt 193 in such a manner as to cover the upper surface of the main body unit section 161 of the main body unit 150 and the head 189 of the operative screw member 153. As described above, as the opening 192 is formed at the regulating plate member 154, the hexagonal hole 188 at the head 189 of the operative screw member 153 is exposed to the outside.

While the wedge projection 182 at the wedge slide member 152 is engaged with the inclined space 178 at the secondary body unit 151, the secondary body unit section 171 of the secondary body unit 151 and the wedge slide member 152 are arranged in the housing hollow section 165 of the main body unit 150. In this state, the wedge slide member 152 is housed movably in the top-bottom direction and the secondary body unit 151 is housed movably in the direction in which the groove 167 of the rod receiving section 162 extends. As a result, as shown in Fig. 10, the one supporting piece 160 of the main body unit 150 and the other supporting piece 170 of the secondary body unit 151 are arranged in such a manner as to face each other.

The male threads 190 of the operative screw member 153 are arranged in the housing hollow section 165 of the main body unit 150 while being threadedly engaged with the female threads at the cylindrical section 181 of the wedge slide member 152. The head 189 of the operative screw member 153 is housed in the housing cavity 164 of the main body unit 150 (main body unit section 161), and the upper surface of the head 189 and the upper surface of the main body unit 150 (main body unit section 161) except the housing cavity 164 are arranged on substantially the same plane. Furthermore, the regulating plate member 154 is arranged in such a manner as to cover the upper surface of the main body unit section 161 of the main body unit 150 and the head 189 of the operative screw member 153, and is fixed with the attachment bolt 193 to the main body unit 150. In this way, the clamp member 50B used in the corrective appliance 1B according to the second embodiment is completed.

For attachment of the clamp member 50B of the corrective appliance 1B according to the second embodiment to the rod receiving section 11 of the screw member 3, a dedicated surgical instrument not shown in the drawings is fitted in the hexagonal hole 188 of the head 189 of the operative screw member 153 and the dedicated surgical instrument is operated to one direction, thereby rotating the operative screw member 153 to the one direction. As a result, as the operative screw member 153 is supported in such a manner as to be unmovable in the top-bottom direction, the wedge slide member 152 is moved upward. This produces wedge effect between the wedge projection 182 at the wedge slide member 152 and the large inclination piece 175A and the small inclination piece 175B in a pair at the secondary body unit section 171 of the secondary body unit 151 to move the other supporting piece 170 of the secondary body unit 151 in a direction of getting father from the one supporting piece 160 of the main body unit 150.

Next, the clamp member 50A is arranged in such a manner as to locate the supporting pieces 160, 170 in a pair thereof at positions on the planar portions 15, 15 in a pair of the rod receiving section 11 of the screw member 3 below the groove 10. At this time, one of the arc-like portions 16 of the rod receiving section 11 of the screw member 3 comes into abutting contact with the screw abutting surface 163 belonging to the main body unit 150 of the clamp member 50A and with the screw abutting surface 172 belonging to the secondary body unit 151.

Next, the dedicated surgical instrument not shown in the drawings is fitted again in the hexagonal hole 188 of the head 189 of the operative screw member 153 and the fitted dedicated surgical instrument is rotated to the opposite direction, thereby rotating the operative screw member 153 to the opposite direction. As a result, the wedge slide member 152 moves downward to produce wedge effect between the wedge projection 182 at the wedge slide member 152 and the large inclination piece 175A and the small inclination piece 175B in a pair at the secondary body unit section 171 of the secondary body unit 151, thereby moving the other supporting piece 170 of the secondary body unit 151 in a direction of getting closer to the one supporting piece 160 of the main body unit 150.

As a result, it becomes possible to firmly clamp the screw member 3 at positions on the planar portions 15, 15 in a pair of the rod receiving section 11 below the groove 10 in such a manner as to clamp the screw member 3 from opposite lateral sides using the supporting pieces 160, 170 in a pair of the clamp member 50B. At this time, as the screw member 3 is sandwiched at the positions on the planar portions 15, 15 in a pair of the rod receiving section 11 of the screw member 3 below the groove 10 using the supporting pieces 160, 170 in a pair of the clamp member 50B, the groove 10 of the screw member 3 (rod receiving section 11) can be exposed to the outside.

The clamp member 50B used in the corrective appliance 1B according to the second embodiment of the present invention includes the supporting pieces 160, 170 in a pair movable closer to and farther from each other. This makes it possible to firmly clamp the screw member 3 at positions on the planar portions 15, 15 in a pair of the rod receiving section 11 below the groove 10 in such a manner as to clamp the screw member 3 from opposite lateral sides using the supporting pieces 160, 170 in a pair. Thus, the groove 10 at the rod receiving section 11 of the screw member 3 can be exposed to the outside. As a result, while spinal deformity is corrected and the corrected state is retained using the clamp member 50B and the auxiliary rod member 51, the rod member 5 can be engaged easily with the groove 10 of each screw member 3.

### Reference Sings List

- 1A, 1B: Corrective appliance
- 2: Spinal deformity correction and fusion system
- 3: Screw member (vertebra fixing tool)
- 4: Hook member (vertebra fixing tool)
- 5: Rod member
- 11: Rod receiving section (head)
- 50A, 50B: Clamp member
- 51: Auxiliary rod member (corrective member)
- 58: Rotary member (ring-like member)
- 63: Groove
- 70: Set screw (fixing tool)
- 92: Stopper pawl section
- 111: Supporting piece (ring-like member)
- 160, 170: Supporting piece

## Claims

1. A corrective appliance (1A, 1B) for assisting in correction and fusion when spinal deformity is subjected to the correction and fusion by a spinal deformity correction and fusion system (2) including a vertebra fixing tool (3, 4) to be fixed to each vertebra of a spine and a rod member (5) to be coupled to the vertebra fixing tool (3, 4), the corrective appliance (1A, 1B) comprising:
a clamp member (50A, 50B) to be attached detachably to the vertebra fixing tool (3, 4) and to be arranged close to the vertebra fixing tool (3, 4); and
a corrective member (51) to be attached detachably to the clamp member (50A, 50B) and to be used for correcting the spinal deformity,
**characterized in that** the clamp member (50A, 50B) includes supporting pieces (111) in a pair movable closer to and farther from each other to support a head of the vertebra fixing tool (3, 4) in such a manner as to sandwich the head.

2. The corrective appliance (1A, 1B) according to claim 1, wherein
the clamp member (50A, 50B) includes a groove (63), and
the corrective member (51) is an auxiliary rod member configured to be engaged with the groove (63) of the clamp member (50A, 50B).

3. The corrective appliance (1A, 1B) according to claim 1 or 2, wherein
the clamp member (50A, 50B) includes a fixing tool (70) for fixing the auxiliary rod member (51) to the groove (63) of the clamp member (50A, 50B).

4. The corrective appliance (1A, 1B) according to claim 3, wherein the clamp member (50A, 50B) includes stopper pawl sections (92) in a pair for sandwiching the head of the vertebra fixing tool (3, 4).

5. The corrective appliance (1A, 1B) according to any one of claims 1 to 4, wherein
the corrective appliance (1A, 1B) is configured as a surgical instrument detachable from the vertebra fixing tool (3, 4) to be taken out of a body while the spinal deformity is corrected and fixed by the spinal deformity correction and fusion system (2).

## Patentansprüche

1. Korrekturvorrichtung (1A, 1B) zur Unterstützung der Korrektur und Fusion, wenn eine Wirbelsäulendeformation einer Korrektur und Fusion durch ein Wirbelsäulendeformationskorrektur- und -fusionssystem (2) unterzogen wird, das ein Wirbelfixierungswerkzeug (3, 4) zur Befestigung an jedem Wirbel einer Wirbelsäule und ein Stabelement (5) zur Verbindung mit dem Wirbelfixierungswerkzeug (3, 4) umfasst, wobei die Korrekturvorrichtung (1A, 1B) umfasst:
ein Klemmelement (50A, 50B), das abnehmbar an dem Wirbelfixierungswerkzeug (3, 4) befestigt und in der Nähe des Wirbelfixierungswerkzeugs (3, 4) angeordnet werden kann; und
ein Korrekturelement (51), das abnehmbar an dem Klemmelement (50A, 50B) befestigt werden kann und zur Korrektur der Wirbelsäulendeformation verwendet werden kann,
**dadurch gekennzeichnet, dass** das Klemmelement (50A, 50B) umfasst
Stützstücke (111) in einem Paar, die näher zueinander und weiter voneinander weg bewegt werden können, um einen Kopf des Wirbelfixierungswerkzeugs (3, 4) so zu stützen, dass der Kopf dazwischen liegt.

2. Korrekturvorrichtung (1A, 1B) nach Anspruch 1, wobei
das Klemmelement (50A, 50B) eine Nut (63) aufweist, und
das Korrekturelement (51) ein Zusatzstabelement ist, das so konfiguriert ist, dass es in die Nut (63) des Klemmelements (50A, 50B) eingreift.

3. Korrekturvorrichtung (1A, 1B) nach Anspruch 1 oder 2, wobei
das Klemmelement (50A, 50B) ein Befestigungswerkzeug (70) zum Befestigen des Zusatzstabelements (51) an der Nut (63) des Klemmelements (50A, 50B) umfasst.

4. Korrekturvorrichtung (1A, 1B) nach Anspruch 3, wobei das Klemmelement (50A, 50B) ein Paar Sperrklinkenabschnitte (92) zum Einklemmen des Kopfes des Wirbelfixierungswerkzeugs (3, 4) aufweist.

5. Korrekturvorrichtung (1A, 1B) nach einem der Ansprüche 1 bis 4, wobei die Korrekturvorrichtung (1A, 1B) als chirurgisches Instrument konfiguriert ist, das vom Wirbelfixierungswerkzeug (3, 4) abnehmbar ist und aus dem Körper entfernt werden kann, während die Wirbelsäulendeformation durch das Wirbelsäulendeformationskorrektur-und -fusionssystem (2) korrigiert und fixiert wird.

## Revendications

1. Appareil correcteur (1A, 1B) pour aider à la correction et à la fusion lorsqu'une déformation de la colonne vertébrale est soumise à une correction et à une fusion par un système (2) de correction et de fusion de déformation de la colonne vertébrale comprenant un outil de fixation sur vertèbre (3, 4) à fixer à chaque vertèbre de la colonne vertébrale et un élément de tige (5) à coupler à l'outil de fixation sur vertèbre (3, 4), l'appareil correcteur (1A, 1B) comprenant :
un élément de serrage (50A, 50B) destiné à être fixé de manière amovible à l'outil de fixation sur vertèbre (3, 4) et à être agencé à proximité de l'outil de fixation sur vertèbre (3, 4), et
un élément correcteur (51) destiné à être fixé de manière amovible à l'élément de serrage (50A, 50B) et servant à corriger la déformation de la colonne vertébrale ;
**caractérisé en ce que** l'élément de serrage (50A, 50B) comprend une paire de pièces de support (111) pouvant être rapprochées et éloignées l'une de l'autre pour soutenir une tête de l'outil de fixation sur vertèbre (3, 4) en enserrant ladite tête.

2. Appareil correcteur (1A, 1B) selon la revendication 1, dans lequel
l'élément de serrage (50A, 50B) comprend une rainure (63), et
l'élément correcteur (51) est un élément de tige auxiliaire conçu pour s'engager dans la rainure (63) de l'élément de serrage (50A, 50B).

3. Appareil correcteur (1A, 1B) selon la revendication 1 ou 2, dans lequel
l'élément de serrage (50A, 50B) comprend un outil de fixation (70) pour fixer l'élément de tige auxiliaire (51) à la rainure (63) de l'élément de serrage (50A, 50B).

4. Appareil correcteur (1A, 1B) selon la revendication 3, dans lequel l'élément de serrage (50A, 50B) comprend une paire de sections de cliquet d'arrêt (92) enserrant la tête de l'outil de fixation sur vertèbre (3, 4).

5. Appareil correcteur (1A, 1B) selon l'une quelconque des revendications 1 à 4, dans lequel
l'appareil correcteur (1A, 1B) a la conception d'un instrument chirurgical détachable de l'outil de fixation sur vertèbre (3, 4) pour être retiré du corps pendant que la déformation de la colonne vertébrale est corrigée et fixée par le système (2) de correction et de fusion de la déformation de la colonne vertébrale.
